# EUROPEAN PATENT SPECIFICATION

(11) **EP 3 870 073 B1**
(45) Date of publication and mention of the grant of the patent: **15.11.2023**
(21) Application number: 19850834.3
(22) Date of filing: 12.12.2019
(51) Int. Cl.: A61B 10/02

(54) **BIOPSY DEVICE WITH TRANSLATING SHUTTLE VALVE ASSEMBLY**
BIOPSIEVORRICHTUNG MIT VERSCHIEBBARER WECHSELVENTILANORDNUNG
DISPOSITIF DE BIOPSIE COMPRENANT UN ENSEMBLE VALVE SÉLECTRICE À TRANSLATION

(30) Priority: 14.12.2018 US 201862779636 P
(43) Date of publication of application: 01.09.2021
(73) Proprietor: Devicor Medical Products, Inc., Cincinnati, OH 45241 (US)
(72) Inventor: RAMAMURTHY, Sivakumar, Chennai Tamil Nadu 600100 (IN); GANGAPPA, Deepakraj, Bangalore Karnataka 560072 (IN)
(74) Representative: Simmons & Simmons
(86) International application number: PCT/US2019/065953
(87) International publication number: WO 2020/123790

(56) References cited:
- EP-A1- 3 073 933
- US-A1- 2007 239 067
- US-A1- 2010 152 610
- US-A1- 2012 109 007
- US-A1- 2014 378 864
- US-A1- 2015 126 902
- US-A1- 2017 000 467
- US-A1- 2017 367 687

## Description

### BACKGROUND

Biopsy samples have been obtained in a variety of ways in various medical procedures using a variety of devices. Biopsy devices may be used under stereotactic guidance, ultrasound guidance, MRI guidance, PEM guidance, BSGI guidance, or otherwise. For instance, some biopsy devices may be fully operable by a user using a single hand, and with a single insertion, to capture one or more biopsy samples from a patient. In addition, some biopsy devices may be tethered to a vacuum module and/or control module, such as for communication of fluids (e.g., pressurized air, saline, atmospheric air, vacuum, etc.), for communication of power, and/or for communication of commands and the like. Other biopsy devices may be fully or at least partially operable without being tethered or otherwise connected with another device. Other biopsy devices may be fully or at least partially operable without being tethered or otherwise connected with another device.

Merely exemplary biopsy devices are disclosed in U.S. Pat. No. 5,526,822, entitled "Method and Apparatus for Automated Biopsy and Collection of Soft Tissue," issued Jun. 18, 1996; U.S. Pat. No. 6,086,544, entitled "Control Apparatus for an Automated Surgical Biopsy Device," issued Jul. 11, 2000; U.S. Pat. No. 6,626,849, entitled "MRI Compatible Surgical Biopsy Device," issued Sept. 30, 2003; U.S. Pat. No. 7,442,171, entitled "Remote Thumbwheel for a Surgical Biopsy Device," issued Oct. 28, 2008; U.S. Pat. No. 7,854,706, entitled "Clutch and Valving System for Tetherless Biopsy Device," issued Dec. 21, 2010; U.S. Pat. No. 8,206,316, entitled "Tetherless Biopsy Device with Reusable Portion, " issued Jun. 26, 2012; U.S. Pat. No. 8,764,680, entitled "Handheld Biopsy Device with Needle Firing," issued Jul. 1, 2014; U.S. Pat. No. 8,801,742, entitled "Needle Assembly and Blade Assembly for Biopsy Device," issued Aug. 12, 2014; U.S. Pat. No. 9,345,457, entitled "Presentation of Biopsy Sample by Biopsy Device," issued May 24, 2016; U.S. Pat. No. 9,724,074, entitled "Biopsy Device with Translating Valve Assembly," issued Aug. 8, 2017; U.S. Pub. No. 2006/0074345, entitled "Biopsy Apparatus and Method," published Apr. 6, 2006; U.S. Pub. No. 2010/0152610, entitled "Hand Actuated Tetherless Biopsy Device with Pistol Grip," published Jun. 17, 2010; and U.S. Pub. No. 2010/0160819, entitled "Biopsy Device with Central Thumbwheel," published Jun. 24, 2010. WO2015/077699 discloses a biopsy device with a translating valve assembly for sealing apertures in a wall of a manifold.
Further exemplary prior art biopsy devices are disclosed in US2017/000467, US2012/109007, US2015/126902, US2014/378864, US2007/239067, and US2017/567687.

While several systems and methods have been made and used for obtaining a biopsy sample, it is believed that no one prior to the inventors has made or used the invention described in the appended claims.
The invention is defined by the appended claims.

### BRIEF DESCRIPTION OF THE FIGURES

While the specification concludes with claims which particularly point out and distinctly claim the biopsy device, it is believed the present biopsy device will be better understood from the following description of certain examples taken in conjunction with the accompanying drawings, in which like reference numerals identify the same elements and in which:
FIG. 1 depicts a perspective view of an exemplary biopsy device;
FIG. 2 depicts a perspective view of the biopsy device of FIG. 1, showing a holster detached from a probe;
FIG. 3 depicts a schematic view of exemplary electrical and/or electromechanical components of the holster of FIG. 2;
FIG. 4 depicts an exploded perspective view of the probe of FIG. 2;
FIG. 5 depicts a perspective view of an exemplary needle assembly and associated components of the probe of FIG. 2;
FIG. 6 depicts a cross-sectional view of the needle assembly of FIG. 5, taken along line 6-6 of FIG. 5;
FIG. 7A depicts a cross-sectional perspective view of the distal end of the needle assembly of FIG. 5, taken along line 7-7 of FIG. 6, showing the cutter in a closed position;
FIG. 7B depicts a cross-sectional perspective view of the distal end of the needle assembly of FIG. 5, taken along line 7-7 of FIG. 6, showing the cutter in an open position;
FIG. 7C depicts a cross-sectional perspective view of the distal end of the needle assembly of FIG. 5, taken along line 7-7 of FIG. 6, showing the cutter in a partially open position;
FIG. 8 depicts an exploded perspective view of the needle assembly of FIG. 5;
FIG. 9 depicts a side cross-sectional perspective view of valve components of the needle assembly of FIG. 5;
FIG. 10 depicts a perspective view of a spool body of the valve components of FIG. 9, oriented with a distal end facing away;
FIG. 11 depicts a perspective view of the spool body of FIG. 10, oriented with a proximal end facing away;
FIG. 12 depicts a cross-sectional view of the spool body of FIG. 10, within the cutter of FIG. 7A disposed within the spool body;
FIG. 13A depicts a cross-sectional perspective view taken along the side of the exemplary needle assembly showing the cutter and the valve assembly in a venting position corresponding to the closed cutter position depicted in FIG. 7A;
FIG. 13B depicts a cross-sectional perspective view taken along the side of the exemplary needle assembly showing the cutter and the valve assembly in a non-venting position corresponding to the opened cutter position depicted in FIG. 7B; and
FIG. 14 depicts a graph showing the relationship between valve state and cutter position.

### DETAILED DESCRIPTION

The following description of certain examples of the biopsy device should not be used to limit the scope of the present biopsy device. Other examples, features, aspects, embodiments, and advantages of the biopsy device will become apparent to those skilled in the art from the following description, which is by way of illustration, one of the best modes contemplated for carrying out the biopsy device. As will be realized, the biopsy device is capable of other different and obvious aspects, falling within the scope of the invention as defined by the claims. Accordingly, the drawings and descriptions should be regarded as illustrative in nature and not restrictive.

### I. Overview of Exemplary Biopsy Device

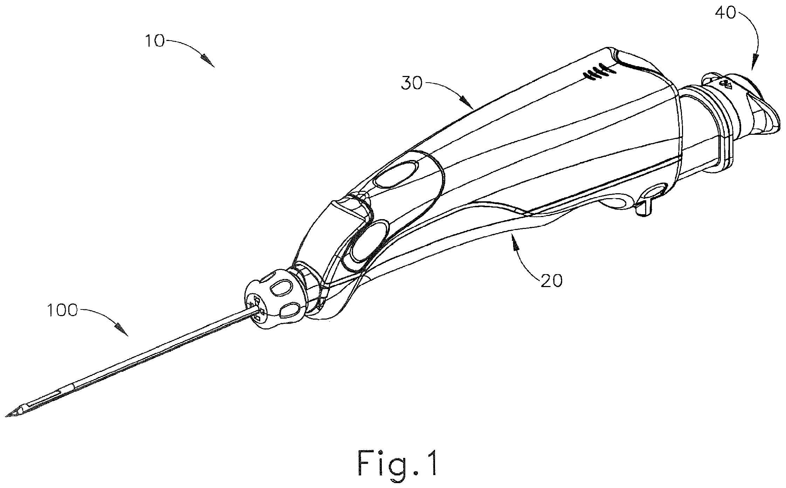

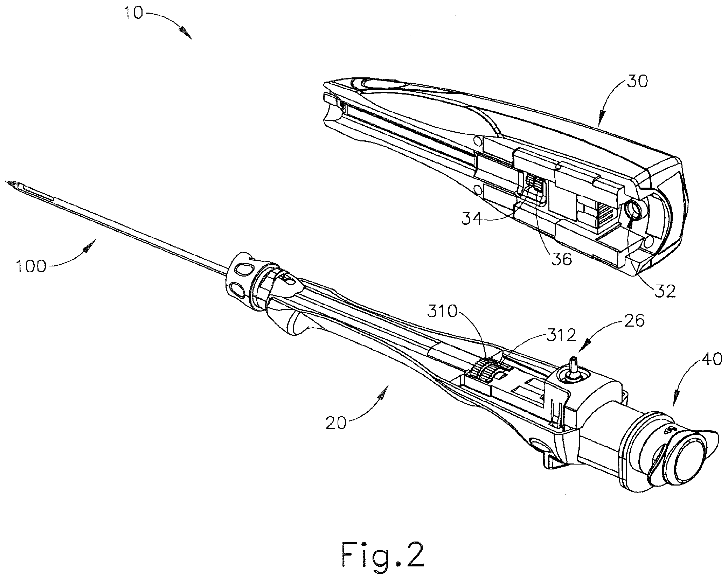

FIG. 1 shows an exemplary biopsy device (10), comprising a probe (20) and a holster (30). Probe (20) comprises a needle assembly (100) that at least partially extends distally from a casing of probe (20). Needle assembly (100) is insertable into a patient's tissue to obtain tissue samples as will be described below. Biopsy device (10) further comprises a tissue sample holder (40) into which the tissue samples are deposited. By way of example only, probe (20) may be a disposable component and holster (30) may be a reusable component to which probe (20) may be coupled, as is shown in FIG. 2. Use of the term "holster" herein should not be read as requiring any portion of probe (20) to be inserted into any portion of holster (30). Indeed, in one configuration for biopsy device (10), probe (20) may simply be positioned atop holster (30). Alternatively, a portion of probe (20) may be inserted into holster (30) to secure probe (20) to holster (30). In yet another configuration, a portion of holster (30) may be inserted into probe (20). Further still, probe (20) and holster (30) may be integrally formed as a single unit.

In configurations where probe (20) and holster (30) are separable members, a port and/or a seal (32) may be provided on holster (30) to couple with a second port and/or a second seal (26) on probe (20) such that the vacuum produced by a vacuum pump (50) within holster (30) may be fluidly connected to probe (20). Holster (30) may also provide gears (34, 36) which mate to and engage with gears (310, 312) on probe (20). It should be understood that the configuration depicted in FIG. 2 that communicates vacuum and motive force between holster (30) and probe (20) is merely exemplary. In some versions, such configurations may be constructed in accordance with at least some of the teachings of U.S. Patent No. 8,206,316 entitled "Tetherless Biopsy Device with Reusable Portion," issued Jun. 26, 2012; and/or U.S. Pub. No. 2012/0065542, entitled "Biopsy Device Tissue Sample Holder with Removable Tray," published Mar. 15, 2012.

With holster (30) and probe (20) connected, vacuum pump (50) can induce a vacuum within needle assembly (100) via tissue sample holder (40) and a tubular cutter (60). However, it should be understood that vacuum may be provided in other ways. For example, vacuum pump (50) may be independent of holster (30) and probe (20) and may simply be coupled by vacuum tubes to appropriate ports on biopsy device (10). Biopsy device (10) may further be configured in accordance with at least some of the teachings of U.S. Patent No. 8,764,680, entitled "Handheld Biopsy Device with Needle Firing," issued July 1, 2014; and/or U.S. Pub. No. 2012/0065542, entitled "Biopsy Device Tissue Sample Holder with Removable Tray," published Mar. 15, 2012. Other suitable structural and functional combinations for probe (20) and holster (30) will be apparent to one of ordinary skill in the art in view of the teachings herein.

### II. Exemplary Holster

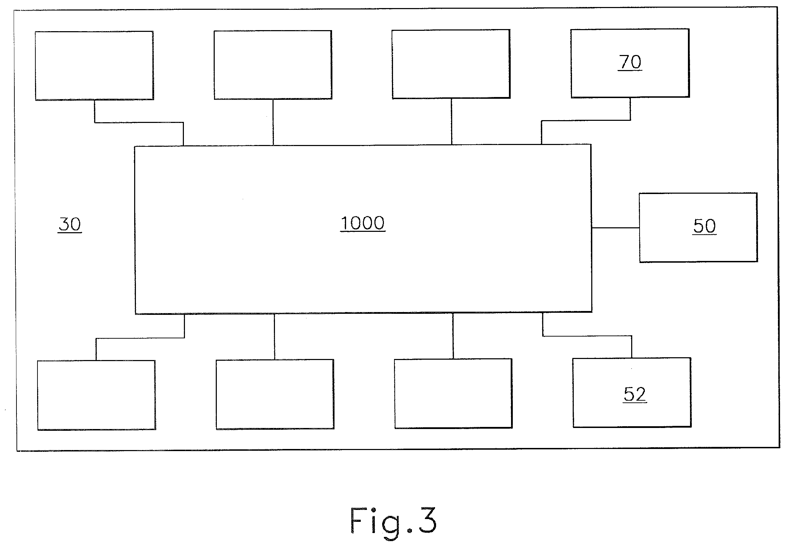

Holster (30), shown schematically in FIG. 3, comprises vacuum pump (50), a motor (70), a control module (1000), a vacuum sensor (52), and any other suitable electrical and/or electromechanical components. Vacuum pump (50) of the present example comprises a conventional diaphragm pump that is mechanically coupled to motor (70). Vacuum sensor (52) is coupled to vacuum pump (50) or along any vacuum path therefrom such that vacuum sensor (52) can determine the level of vacuum created by vacuum pump (50). Vacuum sensor (52) is electrically coupled to control module (1000) so that vacuum sensor (52) may output signals indicative of the vacuum level to control module (1000). In the configuration shown, motor (70) is operable to translate and/or rotate cutter (60), as will be described below, and to activate vacuum pump (50), though this is merely optional and a second motor (not shown) may be provided to run vacuum pump (50). In particular, motor may be coupled to a cutter drive assembly (not shown). Such a cutter drive assembly (not shown) may rotate gears (34, 36) simultaneously. As noted above, gears (34, 36) mesh with gears (310, 312) in probe (20) thus allowing motor (70) to translate and/or rotate cutter (60). Other various configurations for holster (30) may be provided as will be apparent to one of ordinary skill in the art in view of the teachings herein. By way of example only, the cutter drive assembly (not shown) and/or other features of holster (30) may be constructed in accordance with at least some of the teachings of U.S. Patent 8,206,316 entitled "Tetherless Biopsy Device with Reusable Portion," issued Jun. 26, 2012; and/or U.S. Patent No. 8,764,680, entitled "Handheld Biopsy Device with Needle Firing," issued July 1, 2014.

### III. Exemplary Probe

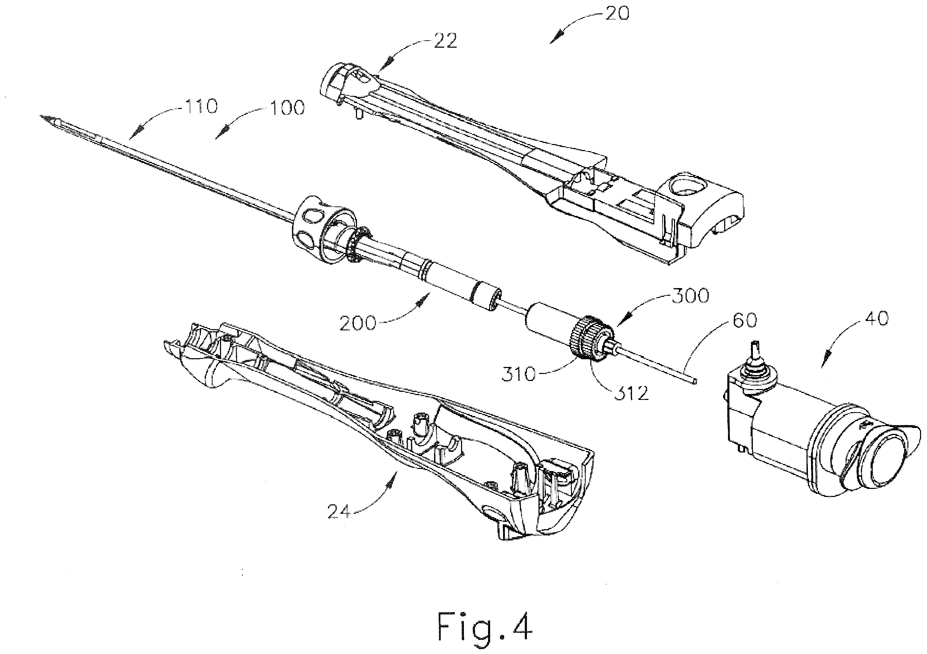

FIG. 4 depicts a partially exploded view of probe (20) showing needle assembly (100), a cutter actuation assembly (300), a probe housing (22, 24) and tissue sample holder (40). Needle assembly (100) comprises a needle portion (110) and a valve assembly (200). As will be described in greater detail below, needle assembly (100) is generally operable to pierce tissue where cutter (60) can be positioned to sever a tissue sample from a patient and transport the tissue sample to tissue sample holder (40). More specifically, the needle portion (110) of needle assembly (100) is inserted into a patient's tissue. Cutter actuation assembly (300) is then operable to selectively actuate cutter (60) to an open position. Once cutter (60) is actuated by cutter actuation assembly (300) into an open position, tissue may be prolapsed into needle portion (110) by means of a vacuum communicated through cutter (60). Cutter (60) may then be selectively actuated by means of cutter actuation assembly (300) into the closed position, severing the prolapsed tissue from the patient. Valve assembly (200) is then operable to selectively vent a portion of needle portion (110) to atmosphere thus creating a pressure differential between proximal and distal ends of the prolapsed tissue. The pressure differential then transports the prolapsed tissue through cutter (60) to tissue sample holder (40).

### A. Exemplary Cutter Actuation Assembly

Cutter actuation assembly (300) comprises a series of gears (310, 312). Gears (310, 312) are configured to translate and/or rotate cutter (60). In the configuration shown, gears (310, 312) are coupled to motor (70) when probe (20) is attached to holster (30). In particular, two gears (310, 312) are controlled by motor (70) such that one gear (310) translates cutter (60) and another gear (312) rotates cutter (60) simultaneously. Other configurations may be provided utilizing different gear (310) arrangements. Moreover, configurations involving additional motors (70) may be used. Various suitable motor (70) and gear (310, 312) combinations will be apparent to one of ordinary skill in the art in view of the teachings herein. Indeed, cutter actuation assembly (300) may be constructed in accordance with at least some of the teachings of U.S. Patent 8,206,316, entitled "Tetherless Biopsy Device with Reusable Portion," issued Jun. 26, 2012.

### B. Exemplary Needle Portion

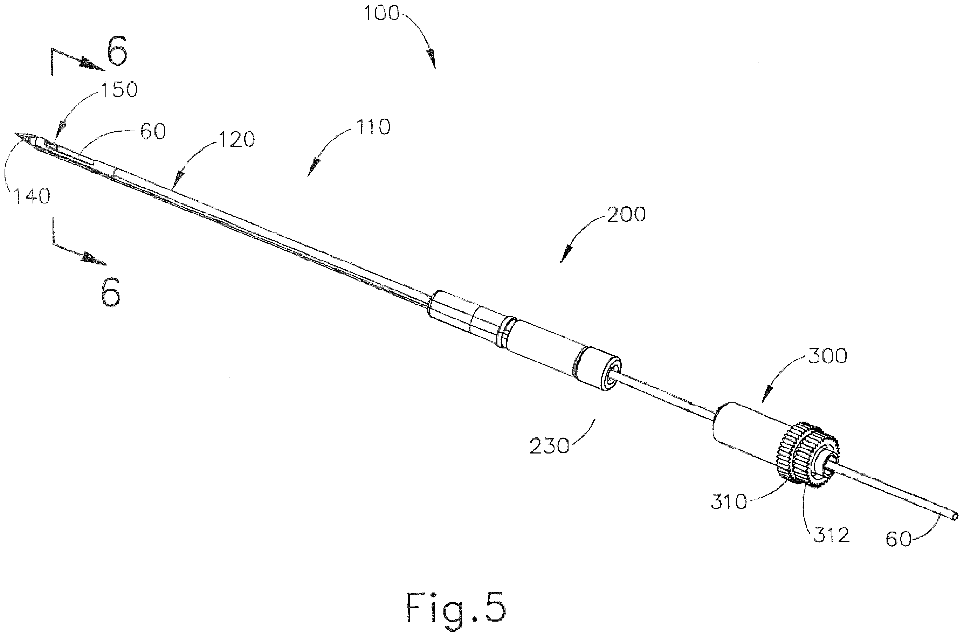

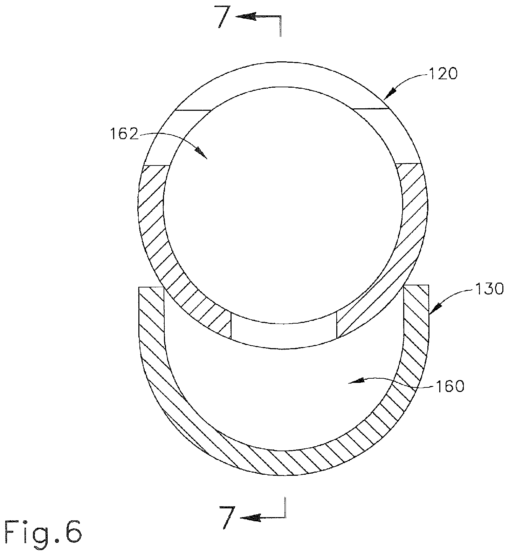

FIGS. 5 through 7 show an exemplary needle portion (110). Needle portion (110) comprises a cannula (120), a partial cannula (130), a tissue piercing tip (140), and a lateral aperture (150). As is shown, cannula (120) is positioned on top of partial cannula (130). Cannula (120) and partial cannula (130) define a first lumen portion (160) and a second lumen portion (162). As is best seen in FIG. 6, cannula (120) is generally circular in shape while partial cannula (130) is semi-circular. Cannula (120) and partial cannula (130) may be coextensive with their proximal end terminating within the valve assembly (200) and their distal end supporting tissue piercing tip (140). Although needle portion (110) is shown as having a generally ovular cross-section, it should be understood that other cross-sectional shapes may be used. Indeed, needle portion (110) may be comprised of only circular tubes thus creating a generally figure eight cross-section. Alternatively, needle portion (110) may be comprised of two square tubes thus creating a generally square cross-section. Yet in other configurations, partial cannula (130) is omitted and only cannula (120) is used to form a generally circular cross-section. In still other configurations, any other suitable shape may be used.

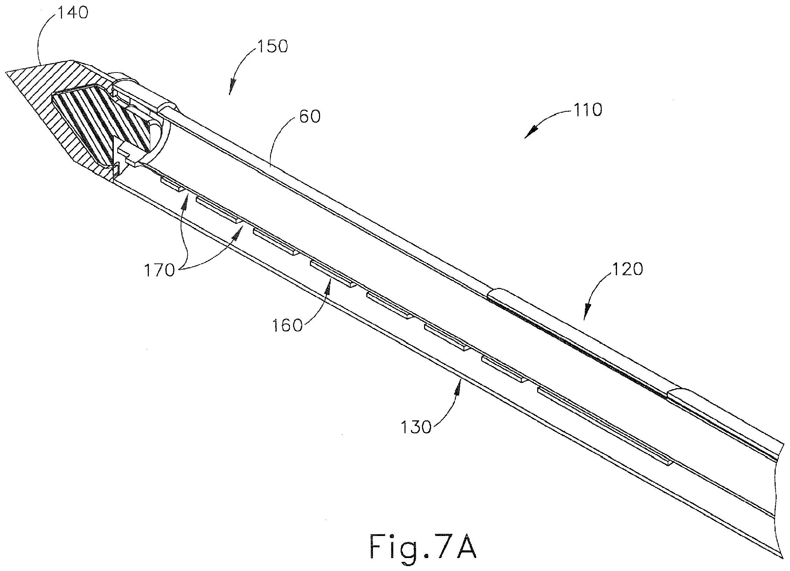

FIGS. 7A to 7C depict needle portion (110) with cutter (60) in various states. In particular, cannula (120) is configured to receive cutter (60) and to permit cutter (60) to translate and rotate within second lumen portion (162). Cannula (120) further comprises lateral aperture (150). Lateral aperture (150) is sized to receive prolapsed tissue during operation of biopsy device (10). The sidewall of cannula (120) opposite lateral aperture (150) comprises a plurality of openings (170) that provide fluid communication between first lumen portion (160) and second lumen portion (162). In the present example, first lumen portion (160) may selectively provide atmospheric air to vent second lumen portion (162) through the plurality of openings (170). Such an atmospheric vent in second lumen portion (162) allows severed tissue to be drawn through cutter (60) and into tissue sample holder (40) under the influence of vacuum from vacuum pump (50).

The series depicted in FIGS. 7A through 7C shows cutter (60) first in a closed position, then in an open position and finally in an intermediate position. Each position depicted may correspond to a particular stage in the tissue sample extraction process. For example, as depicted in FIG. 7A, the cannula (120) may penetrate a patient's tissue when cutter (60) is in a closed position. In the closed position, cutter (60) is in its furthest distal position relative to lateral aperture (150). Thus, cannula (120) may penetrate through tissue smoothly without catching any surrounding tissue that might impede penetration.

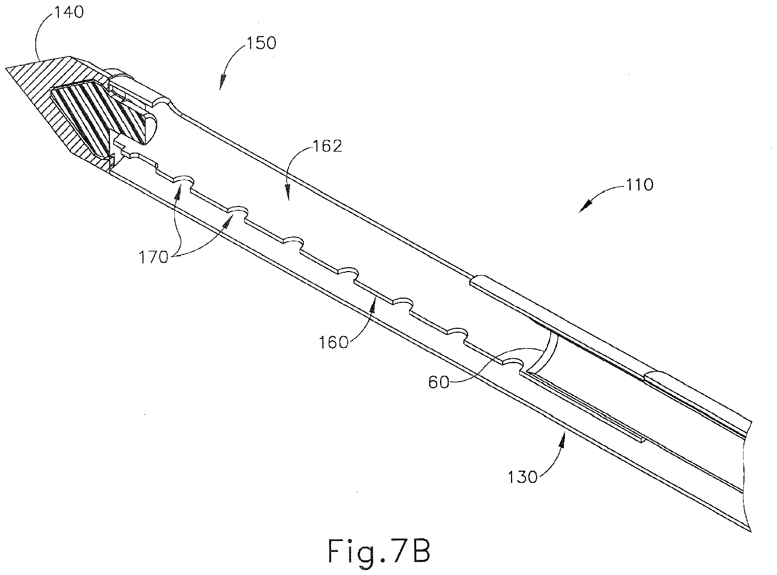

FIG. 7B depicts cutter (60) in the open position, where cutter (60) is in its furthest proximal position relative to lateral aperture (150). This state may, for example, correspond to a position where cannula (120) is oriented inside a patient where a tissue sample may be taken. With cutter (60) in its furthest proximal position relative to lateral aperture (150) a vacuum may be applied to prolapse patient's tissue through lateral aperture (150).

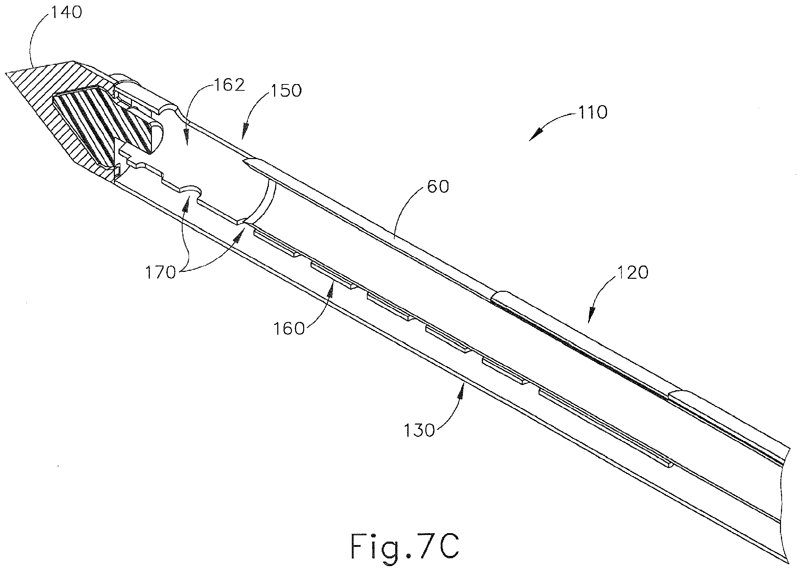

Finally, FIG. 7C depicts cutter (60) in the intermediate position, where cutter (60) is in a position between its furthest distal and proximal positions relative to lateral aperture (150). In this position, cutter (60) may be in a motive state from either a closed position or an open position to a closed or open position, respectively. For example, cutter (60) may move from an open to closed position so that cutter (60) may sever a tissue sample. Alternatively, cutter may move from a closed to open position in order to allow the patient's tissue to prolapse through lateral aperture (150). As will be described in further detail below, these various positions correspond to various pneumatic states of valve assembly (200). It should be understood that the various positions of cutter (60) and the corresponding stages in the tissue extraction process are merely exemplary and other suitable combinations will be apparent to one of ordinary skill in the art from the teachings herein.

Tissue piercing tip (140) is shown as having a generally conical body with a flat blade protruding therefrom. The shape of tissue piercing tip (140) is merely exemplary and many other suitable shapes may be used. For example, tissue piercing tip (140) may be in the shape of a blade protruding from needle portion (110), disregarding the conical body. Still in further variations, the tissue piercing tip (140) may have a flat blade portion of varying shapes and configurations. Other various configurations for tissue piercing tip (140) and for needle portion (110) in general may be provided as will be apparent to one of ordinary skill in the art in view of the teachings herein. By way of example only, needle portion (110) may be constructed in accordance with at least some of the teachings of U.S. Patent No. 8,801,742, entitled "Needle Assembly and Blade Assembly for Biopsy Device," issued August 8, 2014.

### C. Exemplary Valve Assembly

In some instance, it may be desirable to have a compact and simple valve assembly for use in a biopsy device. For instance, as described above, biopsy device (10) is configured as a handheld tetherless biopsy device. In this configuration, it is generally desirable to reduce the size and complexity of parts used in biopsy device (10) to promote the handheld nature of the device. Thus, certain valve assemblies may be desirable if such assemblies have minimal parts and can be readily integrated with other components of biopsy device (10).

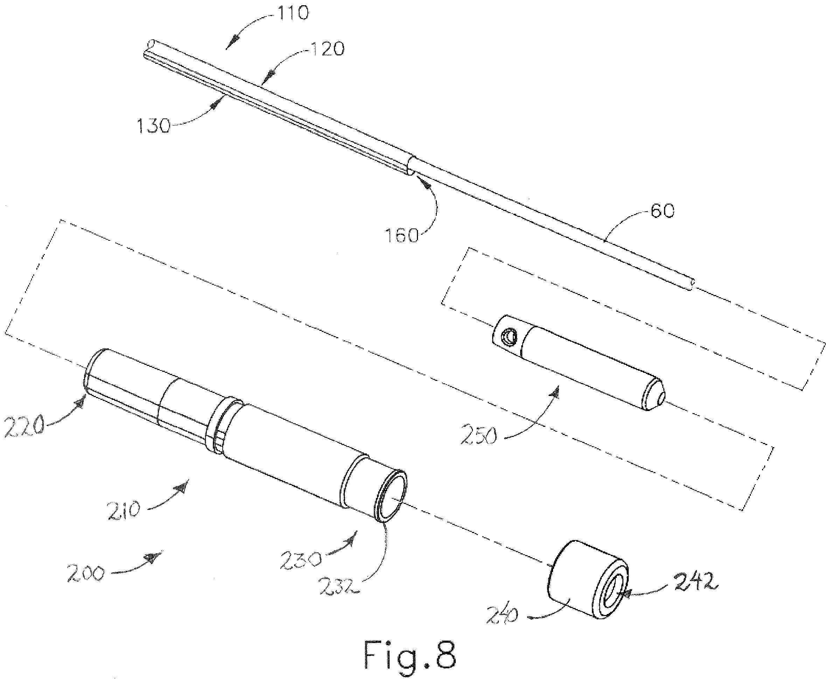

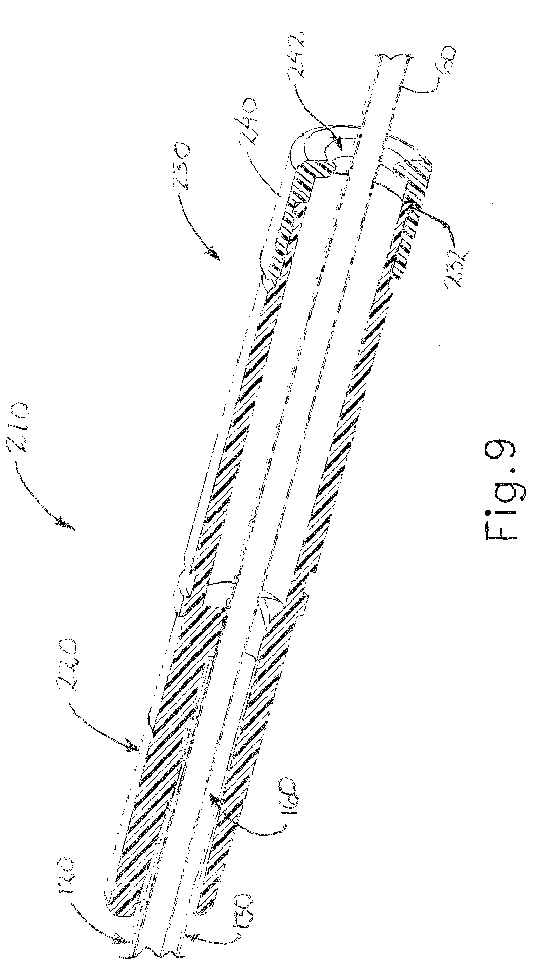

FIG. 8 depicts an exploded view of an exemplary valve assembly (200). Valve assembly (200) comprises a manifold (210), a static seal (240) and a spool body (250) or shuttle valve. In the present example, manifold (210) is configured as a single integrated plastic and/or polycarbonate part. Manifold (210) couples valve assembly (200) to the proximal end of needle portion (110) of needle assembly (100). In particular, manifold (210) comprises a needle coupling end (220) and a venting end (230). As is best seen in FIG. 9, needle coupling end (220) of manifold (210) is configured to receive the proximal end of needle portion (110) of needle assembly (100). In the present example, the coupling is made at the termination of cannula (120) and partial cannula (130). Cutter (60) then continues through valve assembly (200) to tissue sample holder (40). As will be described in more detail below, needle coupling end (220) creates an air tight seal around cannula (120) and partial cannula (130) to permit fluid flow from venting end (230) through first lumen portion (160). Coupling between needle portion (110) and needle coupling end (220) of manifold (210) may be facilitated by any suitable means such as adhesive bonding, a resilient sealing feature, an interference fitting, or a mechanical fastening means.

FIG. 9 shows venting end (230) of manifold (210) in position around cutter (60). Spool body (250) is not shown in FIG. 9 so that the details of venting end (230) may be visible. Venting end (230) extends proximally from needle coupling end (220). In the present example, needle coupling end (220) and venting end (230) are integrally formed as a single unit. In other examples, needle coupling end (220) and venting end (230) may be separate components joined together by any suitable fastening means. Venting end (230) terminates at the proximal end of manifold (210) where static seal (240) is affixed thereto. Venting end (230) of the present example includes a lip (232), flange, channel, or other fastening feature. As will be understood lip (232) is generally configured to engage the interior of static seal (240) to thereby fasten static seal (240) to venting end (230) and to promote sealing between static seal (240) and venting end (230).

Static seal (240) is affixed to the proximal end of manifold (210). Cutter (60) extends through static seal (240). As can be seen, static seal (240) defines a proximal vent opening (242) therein. Vent opening (242) is generally circular and defines a diameter that is oversized relative to the outer diameter of cutter (60). Thus, cutter (60) is free to rotate and translate through static seal (240), while fluid is generally free to pass through vent opening (242) over the exterior of cutter (60). As will be described in greater detail below, this configuration generally allows vent opening (242) to permit the flow of atmospheric air through static seal (240), into venting end (240), and then to first lumen portion (160). However, as will also be described in greater detail below, in some circumstances such fluid flow can be blocked by spool body (250).

The positioning of proximal vent opening (242) at the proximal end and centered within static seal (240) may have certain advantages. For instance, in some configurations of vent assembly (200), one or more vent openings similar to proximal vent opening (242) can be integrated into manifold (210) near the proximate to the center of manifold (210). However, in this configuration, some circumstances can lead to fluid egress from the one or more vent openings as fluid such as blood and/or saline flows from fist lumen portion (160). Repositioning such one or more vent openings to the position of proximal vent opening (242) can be desirable to contain at least some of this fluid egress. For instance, due to the central positioning of proximal vent opening (242) a lip or reservoir is provided by static seal (240) that would not otherwise be present in examples with one or more vent openings in manifold (210).

Static seal (240) is shown as a separate component of valve assembly (200). This allows spool body (250) to be inserted into manifold (210). To promote ease of assembly, in the present example static seal (240) is an elastomeric, rubber, or silicon rubber material such that static seal (240) can be stretched onto venting end (230). As will be described in greater detail below, such materials also can aid in interaction between spool body (250) and static seal (240). It should be understood, however, that static seal (240) can be integrally formed with manifold (210). This may be the case particularly if manifold (210) is comprised of more than one component rather than the unitary design that is shown.

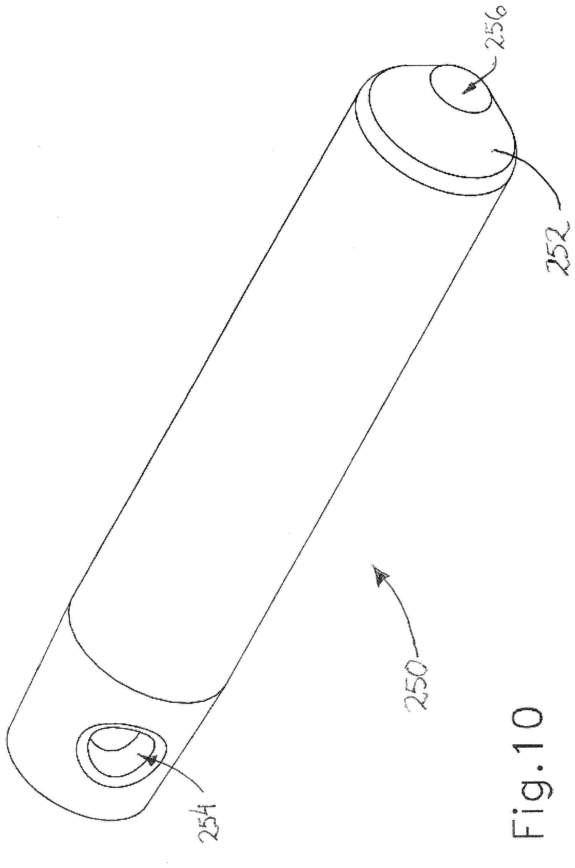

FIGS. 10 through 12 provide detailed views of spool body (250). As can be seen, spool body (250) generally defines an elongate cylindrical body. The proximal end of spool body (250) (e.g., the end extending out of the page in FIG. 10) includes a tapered portion (252) that defines a generally frustoconical shape. As will be described in greater detail below, tapered portion (252) is generally configured to engage static seal (240) to promote sealing between spool body (250) and static seal (240). Additionally, in some examples, tapered portion (252) can also be configured to flex or otherwise displace static seal (240) to further promote sealing. Although tapered portion (252) is shown as having a specific shape in the present example, it should be understood that in other examples the shape of tapered portion (252) can be modified. For instance, in some examples tapered portion (252) may have a greater angle of taper or a lesser angle of taper. In yet other examples, tapered portion (252) can have a concave or convex curvature. Still other configurations will be apparent to those of ordinary skill in the art in view of the teachings herein.

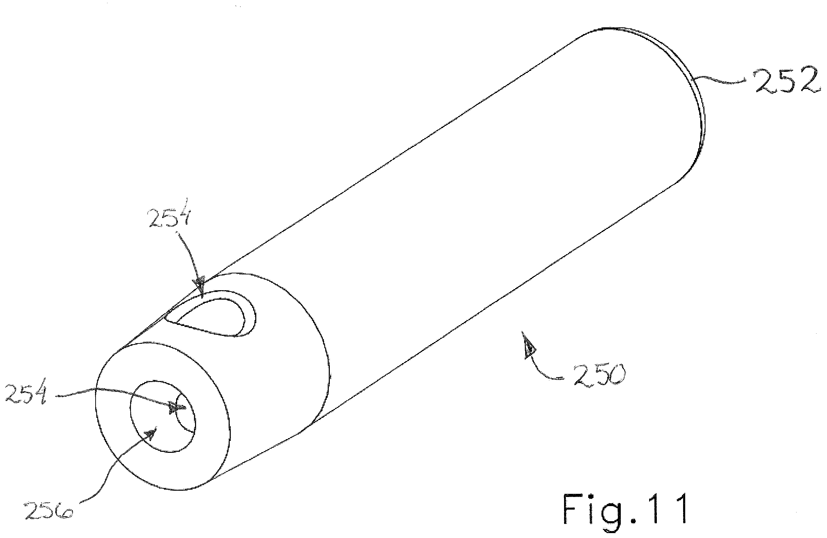

The distal end of spool body (250) (e.g., the end out of the page in FIG. 11) includes one or more fastening openings (254) extending through the body of spool body (250). As can be seen, the present example includes two fastening openings (254), although various alternative numbers can be used. Fastening openings (254) are generally configured to receive glue or adessive to fasten spool body (250) to cutter (60). In other examples, fastening openings (254) can be configured to receive screws or other mechanical fastening features in addition to or in the alternative to glues or adhesives. Further still, fastening openings (254) may even be omitted entirely and replaced with some other means of securing spool body (250) to cutter (60) as will be apparent to one of ordinary skill in the art in light of the teachings herein.

Spool body (250) further includes a lumen (256) extending entirely though the body of spool body (250) from the proximal end to the distal end. Lumen (256) is generally cylindrical in shape with a diameter generally corresponding to the outer diameter of cutter (60). This configuration generally permits lumen (256) to receive cutter (60) while forming a fluid tight seal between cutter (60) and spool body (250). As will be understood, this configuration generally permits spool body (250) to seal first lumen portion (160) relative to static seal (240) during certain stages of operation.

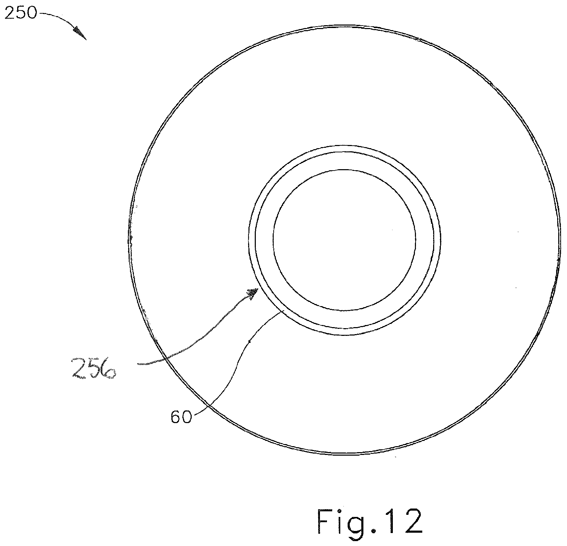

As can best be seen in FIG. 12, when assembled, spool body (250) is positioned on cutter (60) coaxially with cutter (60). Additionally, spool body (250) abuts the outer surface of cutter (60) such that no gap is formed between the interior of spool body (250) and cutter (60). As described above, fastening openings (254) permit glue or adhesive to be placed to secure spool body (250) to cutter (60). Thus, it should be understood that in operation, movement of cutter (60) is immediately transferred to spool body (250) such that spool body (250) and cutter (60) move in unison at all times. Accordingly, spool body (250) may rotate and translate as cutter (60) translates and rotates.

### IV. Exemplary Pneumatic States

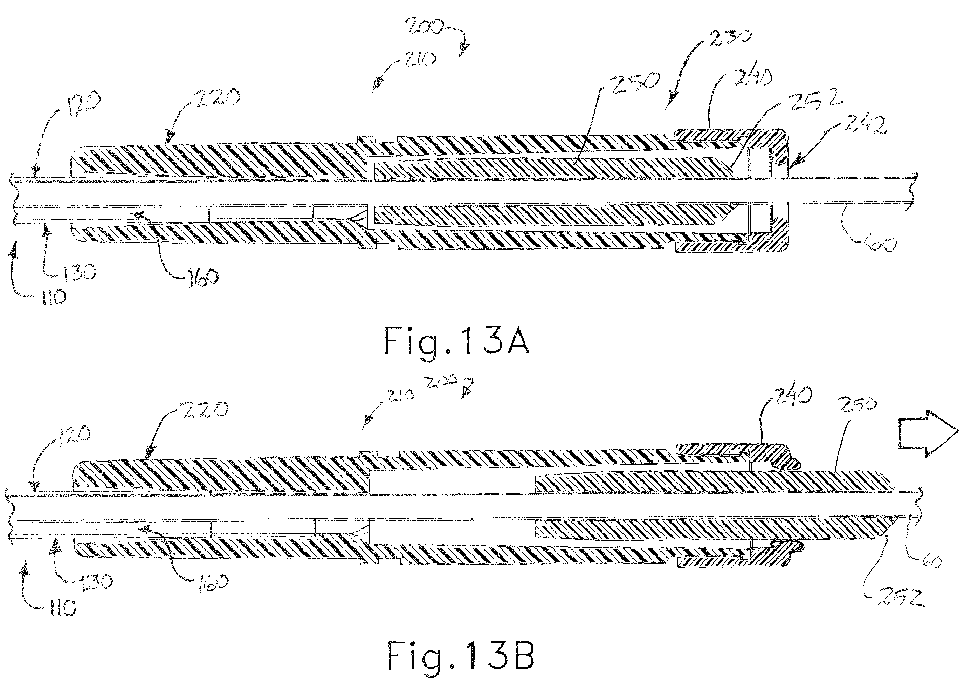

FIGS. 13A to 13B show spool body (250) in various exemplary pneumatic states. In FIG. 13A, spool body (250) is shown in a venting state. In the venting state spool body (250) is in its furthest distal position relative to manifold (210). As was described above, spool body (250) is attached to cutter (60). Accordingly, the venting state corresponds to cutter (60) being in its furthest distal position relative to lateral aperture (150), as can be seen in FIG. 7A. When spool body (250) is positioned in its furthest distal position relative to manifold (210), proximal vent opening (242) permits fluid communication of atmospheric air through static seal (240), into manifold (210), and around an exterior surface of spool body (250) to first lumen portion (160). Spool body (250) thus provides a clear fluid path between proximal vent opening (242) and first lumen portion (160). Fluid communication of atmospheric air to first lumen portion (160) correspondingly allows negative pressure to exist behind a severed tissue sample inside cutter (60) at the distal end of cutter (60). Accordingly, when a vacuum is applied to cutter (60) a severed tissue sample may be transported proximally through cutter (60) to tissue sample holder (40).

Also, when spool body (250) is positioned in its furthest distal position relative to manifold (210), spool body (250) can function as a hard stop to prevent distal translation of cutter (60). As can be seen in FIG. 13A, spool body (250) can engage certain features inside manifold (210) such as an inner step to prevent further distal translation of spool body (250) within manifold. Although this generally prevents further distal translation of cutter (60), it should be understood that at least some rotation of cutter (60) may continue for a predetermined period of time - a configuration known as "free-wheeling."

FIG. 13B depicts spool body (250) in a non-venting state. In the non-venting state, spool body (250) is in its furthest proximal portion relative to manifold (210). In the furthest proximal position, spool body (250) is positioned to seal proximal vent opening (242) in static seal (240). In particular, spool body (250) generally occupies the entire open space between static seal (240) and cutter (60) to thereby seal manifold (210) and first lumen portion (160). In this configuration, the engagement between spool body (250) and static seal (240) to provide sealing of first lumen portion (160) can provide a more robust valve assembly (200). For instance, in some versions of valve assembly (200) spool body (250) includes one or more O-rings to seal against the interior of manifold (210) relative to one or more vent openings in manifold (210). In this configuration, engagement between O-rings and manifold (210) can cause O-rings to degrade over time. However, in the present configuration with proximal vent opening (242) in static seal (240), O-rings can be omitted entirely, thereby preventing degradation and improving robustness. In addition, removal of O-rings can provide more flexibility in materials for manifold (210). For instance, in versions of valve assembly that use O-rings, at lest a portion of manifold (210) is generally constructed of metal to reduce friction between O-rings and manifold (210). However, in the present configuration with O-rings removed, manifold (210) can comprise a unitary polycarbonate part formed by injection molding.

In the present example, this sealing configuration includes at least some displacement of static seal (240) to promote a consistent seal between static seal (240) and spool body (250). However, it should be understood that in other examples alternative configurations can be used. For instance, in other examples static seal (240) can be comprised of a generally rigid material, while spool body (250) can be comprised of an elastomeric material. Thus, in some examples a portion of spool body (250) can be displaced instead of a portion of static seal (240). In yet other examples, neither static seal (240) nor spool body (250) can be displaced, instead relying on a compression fit between the two for sealing. In still other examples, both static seal (240) and spool body (250) can be displaced relative to each other.

FIG. 13B depicts a non-venting state that corresponds to an open state of cutter (60). As can be best seen in FIG. 7B, the open state of cutter (60) corresponds to cutter (60) being disposed in its furthest proximal position relative to lateral aperture (150). In the open state of cutter (60), there is no fluid communication of atmospheric air through first lumen portion (160). In other words, spool body (250) seals first lumen portion (160) relative to atmosphere at this stage. Thus, when a vacuum is applied to cutter (60), tissue may be prolapsed through lateral aperture (150).

Between the positions shown in FIG. 13A and 13B, spool body (250) can travel through an intermediate non-venting state. In the present example, spool body (250) generally defines a longitudinal length. Because of this longitudinal length, and because spool body (250) translates with cutter (60), spool body (250) can remain engaged with static seal (240) and in the non-venting state as cutter (60) translates between its furthest proximal position to its furthest distal position relative to lateral aperture (150). Valve assembly (200) can remain in this non-venting state until cutter (60), for example, reaches the position shown in FIG. 7C. It should be understood that the particular transition point between the venting state and the non-venting state of valve assembly (200) can be varied based on a variety of factors. For instance, some factors that can influence the transition point are the particular axial position of spool body (250) on cutter (60) and/or the longitudinal length of spool body (250). Additionally, the amount of time valve assembly (200) remains in the non-venting state can be readily modified by modifying the longitudinal length of spool body (250) (provided the cutter translation speed remains the same). For instance, the longitudinal length may be less if it is desirable to vent to atmosphere when cutter (60) is in a more proximal position relative to lateral aperture (150). Alternatively, needle portion (110) may be of a different configuration necessitating a different distance such that the transition between a non-venting state and a venting state remains the same relative to cutter (60) position. Other configurations involving a different distance between spool body (250), the positioning of spool body (250), and the longitudinal length of spool body (250) will be apparent to one of ordinary skill in the art in view of the teachings herein.

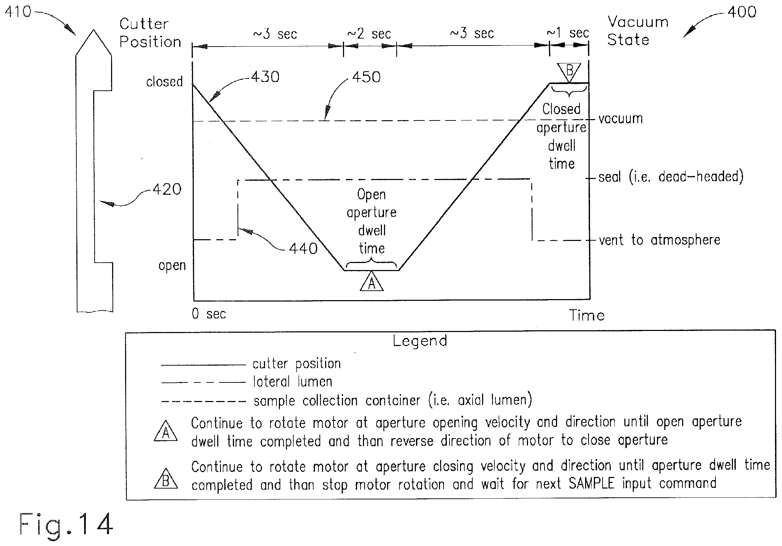

FIG. 14 depicts an exemplary pneumatic algorithm (400) that may be carried out during the use of biopsy device (10). In particular, FIG. 14 shows movements of cutter (60) in relation to cannula (120), which is represented by graphical representation (410) including a graphical representation (420) of lateral aperture (150). Movement of cutter (60) is shown in line (430) for a full range of travel for cutter (60). Line (440) represents pneumatic states of valve assembly (200) during the tissue extraction process; thereby indicating the pneumatic states of first lumen portion (160). The pneumatic state of second lumen portion (162) inside cutter (60) is shown by line (450). As can be seen, vacuum is applied to second lumen portion (162) constantly throughout the tissue extraction process. The term "dead head" in FIG. 14 is intended to mean that the corresponding first lumen portion (160) is sealed relative to atmosphere, and that vacuum is not permitted to freely flow from second lumen portion (162) to first lumen portion (160) during that stage.

As shown in FIG. 14, cutter (60) begins in the closed position which is shown in FIG. 7A. In this position, line (440) of FIG. 14 shows that valve assembly (200) is vented to atmosphere and line (450) shows second lumen portion (162) having a vacuum applied thereto. The corresponding position of valve assembly (200) can be seen in FIG. 13A.

As cutter (60) translates proximally from a closed state toward an open state, as shown by line (430) of FIG. 14 and as depicted in FIG. 7C, line (440) shows that valve assembly (200) correspondingly shifts to a "dead head" state where first lumen portion (160) is sealed to atmosphere. In the present example, this transition occurs when cutter (60) is located at a position to lateral aperture (150) where lateral aperture (150) is effectively approximately 24% open. However, it should be understood, that such a transition may occur at other cutter (60) positions relative to lateral aperture (150).

Once cutter (60) reaches an open position, as depicted in FIG. 7B, line (430) in FIG. 14 shows that cutter (60) may remain open for an open aperture dwell time (460). During open aperture dwell time (460), first lumen portion (160) is sealed relative to atmosphere. The corresponding position of valve assembly (200) is best seen in FIG. 13B. However, as indicated by line (450) a vacuum remains applied to second lumen portion (162) inside cutter (60). Thus, vacuum may travel through lateral aperture (150) via second lumen portion (162) such that tissue may be prolapsed through lateral aperture (150). In some examples, pneumatic algorithm (400) may include continued rotation of motor (70) at the same velocity as when cutter (60) was being opened. In such examples, motor (70) may be decoupled from a cutter drivetrain for the duration of open aperture dwell time (460). Yet, motor (70) may remain coupled to vacuum pump (50) to supply vacuum as indicated by line (450).

Line (430) of FIG. 14 next depicts cutter (60) shifting distally from an open position toward a closed position as shown in FIG. 7C. As cutter (60) translates distally, severing the prolapsed tissue, line (440) of FIG. 14 shows vent assembly (200) shifting from a dead head state to a venting state. As described above, this transition occurs when cutter (60) is located at a position to lateral aperture (150) where lateral aperture (150) is effectively approximately 13% open. However, it should likewise be understood that this position may be varied in other versions.

Finally, line (430) of FIG. 14 shows cutter (60) back in a closed position, as depicted in FIG. 7A, where it will remain for a closed aperture dwell time (470). In this state, line (440) of FIG. 14 depicts valve assembly (200) in a venting state as is shown in FIG. 13A. Thus, first lumen (160) may vent to atmosphere to create a pressure differential suitable for proximal movement of a severed tissue sample through cutter (60) and into tissue sample holder (40). As similarly described above with respect to open aperture dwell time (460), closed aperture dwell time (470) may include continued rotation of motor (70). However, unlike with open aperture dwell time (460), motor (70) may continue to rotate at the same velocity as when cutter (60) was being closed. It should be understood that during such rotation of motor (70), motor (70) may be decoupled from the cutter drivetrain for the duration of closed aperture dwell time (470). However, motor (70) may remain coupled to vacuum pump (50) to supply vacuum as indicated by line (450). Once closed aperture dwell time (470) has expired, the process described above may then be repeated as necessary to obtain the desired number of tissue samples. Of course, the various states described above are merely exemplary and other relationships between cutter (60) position, vent state, and vacuum will be apparent to one of ordinary skill in the art in view of the teachings herein.

Embodiments of the present invention have application in conventional endoscopic and open surgical instrumentation as well as application in robotic-assisted surgery.

Embodiments of the devices disclosed herein can be designed to be disposed of after a single use, or they can be designed to be used multiple times. Embodiments may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, embodiments described herein may be processed before a procedure. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A biopsy device (10), comprising:
(a) a body (20);
(b) a needle (100) extending distally relative to the body (20), wherein the needle (100) defines a first lumen (162) and a second lumen (160), wherein the needle (100) includes an opening (170) fluidly coupling the first lumen with the second lumen;
(c) a cutter (60), wherein the cutter is configured to translate relative to the needle (100) to sever tissue; and
(d) a valve assembly (200), the valve assembly (200) including:
(i) a manifold (210) including a proximal vent opening (242), and
(ii) a spool body (250) configured to move relative to the proximal vent opening (242) between a first position and a second position, wherein the second lumen (160) of the needle is coupled to the proximal vent opening (242) when the spool body (250) is in the first position, wherein the second lumen (160) is sealed relative to the proximal vent opening (242) when the spool body (250) is in the second position, wherein the spool body (250) is configured to transition between the first and second position by moving in proportion to translation of the cutter (60), and
**characterized in that** the spool body (250) is receivable within the proximal vent opening (242) to seal the second lumen (160) relative to the proximal vent opening (242).

2. The biopsy device of claim 1, wherein the needle (100) further defines a transverse tissue receiving aperture (150), wherein the transverse tissue receiving aperture (150) opens into the first lumen (162), wherein the cutter (60) is operable to sever tissue protruding through the tissue receiving aperture (150).

3. The biopsy device of claims 1 or 2, wherein the first lumen (162) extends along a first longitudinal axis, wherein the second lumen (160) extends along a second longitudinal axis, wherein the cutter (60) is configured to translate along the first axis.

4. The biopsy device of any one or more of claims 1 through 3, wherein the cutter (60) is positioned in the first lumen (162).

5. The biopsy device of any one or more of claims 1 through 4, wherein the spool body (250) is movable along an axis defined by the cutter (60) or the first lumen (162).

6. The biopsy device of any one or more of claims 1 through 5, wherein the spool body (250) is coupled to the cutter (60) such that translation of the cutter (60) directly corresponds to movement of the spool body (250) along an axis defined by the cutter (60).

7. The biopsy device of any one or more of claims 1 through 6, wherein the cutter (60) is translatable between a first position and a second position, wherein the first position of the cutter (60) corresponds to the spool body (250) being in the first position, wherein the second position of the cutter (60) corresponds to the spool body (250) being in the second position.

8. The biopsy device of claim 7, wherein the cutter (60) is fully advanced distally relative to the needle (100) when the cutter (60) is in the first position, wherein the cutter (60) is fully advanced proximally relative to the needle (100) when the cutter (60) is in the second position.

9. The biopsy device of any one or more of claims 1 through 8, wherein the proximal vent opening (242) is defined by the manifold (210).

10. The biopsy device of any one or more of claims 1 through 8, wherein the valve assembly (200) further includes a seal (240), wherein the seal (240) defines the proximal vent opening (242) and is secured to the manifold (210).

11. The biopsy device of any one or more of claims 1 through 10, wherein the manifold (210) is fixedly secured to the distal end of the body (20), wherein the manifold (210) extends proximally from the distal end of the body (20), wherein the needle (100) extends distally from the manifold (210), wherein the needle (100) is fluidly sealed relative to the manifold (210).

12. The biopsy device of any one or more of claims 1 through 11, wherein the spool body (250) is disposed within the manifold (210), wherein the spool body (250) is slidable within at least a portion of the manifold (210) relative to the proximal vent opening (242).

13. The biopsy device of any one or more of claims 1 through 12, wherein the spool body (250) defines an open space between an outer surface of the spool body (250) and an interior surface of the manifold (210), wherein the open space is configured to permit the flow of atmospheric air around the spool body (250).

14. The biopsy device of any one or more of claims 1 through 13, wherein the spool body (250) defines an inner lumen for receiving the cutter (60), wherein the spool body (250) is configured to receive the cutter (60) such that an inner surface of the spool body (250) directly abuts the cutter (60).

15. The biopsy device of any one or more of claims 1 through 14, wherein the spool body (250) is configured to plug the proximal vent opening (242) to seal the second lumen (160) relative to the proximal vent opening (242).

16. The biopsy device of claim 10, wherein the seal (240) is located at a proximal end of the manifold (210) and the cutter (60) extends through the seal (240), wherein the proximal vent opening (242) is defined between the cutter (60) and the seal (240).

## Patentansprüche

1. Biopsievorrichtung (10), umfassend:
(a) einen Körper (20);
(b) eine Nadel (100), die sich distal relativ zu dem Körper (20) erstreckt, wobei die Nadel (100) ein erstes Lumen (162) und ein zweites Lumen (160) definiert, wobei die Nadel (100) eine Öffnung (170) beinhaltet, die das erste Lumen mit dem zweiten Lumen fluidmäßig koppelt;
(c) einen Cutter (60), wobei der Cutter so konfiguriert ist, dass er sich relativ zur Nadel (100) verschiebt, um Gewebe zu durchtrennen; und
(d) eine Ventilanordnung (200), wobei die Ventilanordnung (200) Folgendes beinhaltet:
(i) einen Verteiler (210), der eine proximale Entlüftungsöffnung (242) beinhaltet, und
(ii) einen Spulenkörper (250), der so konfiguriert ist, dass er sich relativ zu der proximalen Entlüftungsöffnung (242) zwischen einer ersten Position und einer zweiten Position bewegt, wobei das zweite Lumen (160) der Nadel mit der proximalen Entlüftungsöffnung (242) gekoppelt ist, wenn sich der Spulenkörper (250) in der ersten Position befindet, wobei das zweite Lumen (160) relativ zu der proximalen Entlüftungsöffnung (242) abgedichtet ist, wenn sich der Spulenkörper (250) in der zweiten Position befindet, wobei der Spulenkörper (250) so konfiguriert ist, dass er zwischen der ersten und der zweiten Position übergeht, indem er sich proportional zur Verschiebung des Cutters (60) bewegt, und
**dadurch gekennzeichnet, dass** der Spulenkörper (250) innerhalb der proximalen Entlüftungsöffnung (242) aufnehmbar ist, um das zweite Lumen (160) relativ zur proximalen Entlüftungsöffnung (242) abzudichten.

2. Biopsievorrichtung nach Anspruch 1, wobei die Nadel (100) weiter eine quer verlaufende Gewebeaufnahmeöffnung (150) definiert, wobei sich die quer verlaufende Gewebeaufnahmeöffnung (150) in das erste Lumen (162) hinein öffnet, wobei der Cutter (60) betreibbar ist, um durch die Gewebeaufnahmeöffnung (150) vorstehendes Gewebe abzutrennen.

3. Biopsievorrichtung nach Anspruch 1 oder 2, wobei sich das erste Lumen (162) entlang einer ersten Längsachse erstreckt, wobei sich das zweite Lumen (160) entlang einer zweiten Längsachse erstreckt, wobei der Cutter (60) so konfiguriert ist, dass er sich entlang der ersten Achse verschiebt.

4. Biopsievorrichtung nach einem oder mehreren der Ansprüche 1 bis 3, wobei der Cutter (60) in dem ersten Lumen (162) positioniert ist.

5. Biopsievorrichtung nach einem oder mehreren der Ansprüche 1 bis 4, wobei der Spulenkörper (250) entlang einer durch den Cutter (60) oder das erste Lumen (162) definierten Achse beweglich ist.

6. Biopsievorrichtung nach einem oder mehreren der Ansprüche 1 bis 5, wobei der Spulenkörper (250) mit dem Cutter (60) so gekoppelt ist, dass eine Verschiebung des Cutters (60) direkt einer Bewegung des Spulenkörpers (250) entlang einer durch den Cutter (60) definierten Achse entspricht.

7. Biopsievorrichtung nach einem oder mehreren der Ansprüche 1 bis 6, wobei der Cutter (60) zwischen einer ersten Position und einer zweiten Position verschiebbar ist, wobei die erste Position des Cutters (60) dem Spulenkörper (250) in der ersten Position entspricht, wobei die zweite Position des Cutters (60) dem Spulenkörper (250) in der zweiten Position entspricht.

8. Biopsievorrichtung nach Anspruch 7, wobei der Cutter (60) vollständig distal relativ zur Nadel (100) vorgeschoben ist, wenn sich der Cutter (60) in der ersten Position befindet, wobei der Cutter (60) vollständig proximal relativ zur Nadel (100) vorgeschoben ist, wenn sich der Cutter (60) in der zweiten Position befindet.

9. Biopsievorrichtung nach einem oder mehreren der Ansprüche 1 bis 8, wobei die proximale Entlüftungsöffnung (242) durch den Verteiler (210) definiert ist.

10. Biopsievorrichtung nach einem oder mehreren der Ansprüche 1 bis 8, wobei die Ventilanordnung (200) weiter eine Dichtung (240) beinhaltet, wobei die Dichtung (240) die proximale Entlüftungsöffnung (242) definiert und am Verteiler (210) befestigt ist.

11. Biopsievorrichtung nach einem oder mehreren der Ansprüche 1 bis 10, wobei der Verteiler (210) fest an dem distalen Ende des Körpers (20) befestigt ist, wobei sich der Verteiler (210) proximal von dem distalen Ende des Körpers (20) erstreckt, wobei sich die Nadel (100) distal von dem Verteiler (210) erstreckt, wobei die Nadel (100) relativ zu dem Verteiler (210) fluiddicht ist.

12. Biopsievorrichtung nach einem oder mehreren der Ansprüche 1 bis 11, wobei der Spulenkörper (250) innerhalb des Verteilers (210) angeordnet ist, wobei der Spulenkörper (250) innerhalb mindestens eines Abschnitts des Verteilers (210) relativ zu der proximalen Entlüftungsöffnung (242) verschiebbar ist.

13. Biopsievorrichtung nach einem oder mehreren der Ansprüche 1 bis 12, wobei der Spulenkörper (250) einen offenen Raum zwischen einer Außenfläche des Spulenkörpers (250) und einer Innenfläche des Verteilers (210) definiert, wobei der offene Raum so konfiguriert ist, dass atmosphärische Luft um den Spulenkörper (250) herum strömen kann.

14. Biopsievorrichtung nach einem oder mehreren der Ansprüche 1 bis 13, wobei der Spulenkörper (250) ein inneres Lumen zur Aufnahme des Cutters (60) definiert, wobei der Spulenkörper (250) so konfiguriert ist, dass er den Cutter (60) so aufnimmt, dass eine Innenfläche des Spulenkörpers (250) direkt an den Cutter (60) anstößt.

15. Biopsievorrichtung nach einem oder mehreren der Ansprüche 1 bis 14, wobei der Spulenkörper (250) so konfiguriert ist, dass er die proximale Entlüftungsöffnung (242) verschließt, um das zweite Lumen (160) relativ zu der proximalen Entlüftungsöffnung (242) abzudichten.

16. Biopsievorrichtung nach Anspruch 10, wobei sich die Dichtung (240) an einem proximalen Ende des Verteilers (210) befindet und der Cutter (60) sich durch die Dichtung (240) erstreckt, wobei die proximale Entlüftungsöffnung (242) zwischen dem Cutter (60) und der Dichtung (240) definiert ist.

## Revendications

1. Dispositif de biopsie (10), comprenant :
(a) un corps (20) ;
(b) une aiguille (100) s'étendant de manière distale par rapport au corps (20), dans lequel l'aiguille (100) définit une première lumière (162) et une seconde lumière (160), dans lequel l'aiguille (100) inclut une ouverture (170) couplant fluidiquement la première lumière avec la seconde lumière ;
(c) une lame (60), dans lequel la lame est configurée pour se déplacer en translation par rapport à l'aiguille (100) pour couper le tissu ; et
(d) un ensemble soupape (200), l'ensemble soupape (200) incluant :
(i) un collecteur (210) incluant une ouverture d'aération proximale (242), et
(ii) un corps de bobine (250) configuré pour se déplacer par rapport à l'ouverture d'aération proximale (242) entre une première position et une seconde position, dans lequel la seconde lumière (160) de l'aiguille est couplée à l'ouverture d'aération proximale (242) lorsque le corps de bobine (250) est dans la première position, dans lequel la seconde lumière (160) est obturée de manière étanche par rapport à l'ouverture d'aération proximale (242) lorsque le corps de bobine (250) est dans la seconde position, dans lequel le corps de bobine (250) est configuré pour effectuer une transition entre la première position et la seconde position en se déplaçant proportionnellement à la translation de la lame (60), et
**caractérisé en ce que** le corps de bobine (250) peut être reçu à l'intérieur de l'ouverture d'aération proximale (242) pour obturer de manière étanche la seconde lumière (160) par rapport à l'ouverture d'aération proximale (242).

2. Dispositif de biopsie selon la revendication 1, dans lequel l'aiguille (100) définit en outre un trou transversal de réception de tissu (150), dans lequel le trou transversal de réception de tissu (150) donne sur la première lumière (162), dans lequel la lame (60) peut servir à couper le tissu dépassant du trou de réception de tissu (150).

3. Dispositif de biopsie selon la revendication 1 ou 2, dans lequel la première lumière (162) s'étend le long d'un premier axe longitudinal, dans lequel la seconde lumière (160) s'étend le long d'un second axe longitudinal, dans lequel la lame (60) est configurée pour se déplacer en translation le long du premier axe.

4. Dispositif de biopsie selon l'une quelconque ou plusieurs des revendications 1 à 3, dans lequel la lame (60) est positionnée dans la première lumière (162).

5. Dispositif de biopsie selon l'une quelconque ou plusieurs des revendications 1 à 4, dans lequel le corps de bobine (250) est mobile le long d'un axe défini par la lame (60) ou la première lumière (162).

6. Dispositif de biopsie selon l'une quelconque ou plusieurs des revendications 1 à 5, dans lequel le corps de bobine (250) est couplé à la lame (60) de sorte que la translation de la lame (60) corresponde directement au déplacement du corps de bobine (250) le long d'un axe défini par la lame (60).

7. Dispositif de biopsie selon l'une quelconque ou plusieurs des revendications 1 à 6, dans lequel la lame (60) peut être déplacée en translation entre une première position et une seconde position, dans lequel la première position de la lame (60) correspond au corps de bobine (250) situé dans la première position, dans lequel la seconde position de la lame (60) correspond au corps de bobine (250) situé dans la seconde position.

8. Dispositif de biopsie selon la revendication 7, dans lequel la lame (60) est complètement avancée de manière distale par rapport à l'aiguille (100) lorsque la lame (60) est dans la première position, dans lequel la lame (60) est complètement avancée de manière proximale par rapport à l'aiguille (100) lorsque la lame (60) est dans la seconde position.

9. Dispositif de biopsie selon l'une quelconque ou plusieurs des revendications 1 à 8, dans lequel l'ouverture d'aération proximale (242) est définie par le collecteur (210).

10. Dispositif de biopsie selon l'une quelconque ou plusieurs des revendications 1 à 8, dans lequel l'ensemble soupape (200) inclut en outre un joint (240), dans lequel le joint (240) définit l'ouverture d'aération proximale (242) et est fixé au collecteur (210).

11. Dispositif de biopsie selon l'une quelconque ou plusieurs des revendications 1 à 10, dans lequel le collecteur (210) est fixé à demeure à l'extrémité distale du corps (20), dans lequel le collecteur (210) s'étend de manière proximale depuis l'extrémité distale du corps (20), dans lequel l'aiguille (100) s'étend de manière distale à partir du collecteur (210), dans lequel l'aiguille (100) est obturée de manière étanche aux fluides par rapport au collecteur (210).

12. Dispositif de biopsie selon l'une quelconque ou plusieurs des revendications 1 à 11, dans lequel le corps de bobine (250) est disposé à l'intérieur du collecteur (210), dans lequel le corps de bobine (250) peut coulisser dans au moins une partie du collecteur (210) par rapport à l'ouverture d'aération proximale (242).

13. Dispositif de biopsie selon l'une quelconque ou plusieurs des revendications 1 à 12, dans lequel le corps de bobine (250) définit un espace ouvert entre une surface externe du corps de bobine (250) et une surface intérieure du collecteur (210), dans lequel l'espace ouvert est configuré pour permettre la circulation de l'air atmosphérique autour du corps de bobine (250).

14. Dispositif de biopsie selon l'une quelconque ou plusieurs des revendications 1 à 13, dans lequel le corps de bobine (250) définit une lumière interne pour recevoir la lame (60), dans lequel le corps de bobine (250) est configuré pour recevoir la lame (60) de sorte qu'une surface interne du corps de bobine (250) vienne directement en butée contre la lame (60).

15. Dispositif de biopsie selon l'une quelconque ou plusieurs des revendications 1 à 14, dans lequel le corps de bobine (250) est configuré pour boucher l'ouverture d'aération proximale (242) pour obturer de manière étanche la seconde lumière (160) par rapport à l'ouverture d'aération proximale (242).

16. Dispositif de biopsie selon la revendication 10, dans lequel le joint (240) est situé au niveau d'une extrémité proximale du collecteur (210) et la lame (60) s'étend à travers le joint (240), dans lequel l'ouverture d'aération proximale (242) est définie entre la lame (60) et le joint (240).
